(19) Europäisches Patentamt
European Patent Office
Office européen des brevets

(11) Publication number: **0 101 829**
**B1**

(12) # EUROPEAN PATENT SPECIFICATION

(45) Date of publication of patent specification: **30.12.86**

(21) Application number: **83106341.7**

(22) Date of filing: **29.06.83**

(51) Int. Cl.[4]: **C 07 D 498/06,**
C 07 D 455/04,
A 61 K 31/535, A 61 K 31/47
// (C07D498/06, 265:00,
221:00)

(54) **Tricyclic compounds, a process for their production and pharmaceutical compositions containing said compounds.**

(30) Priority: **29.06.82 JP 112040/82**
**10.12.82 JP 216545/82**

(43) Date of publication of application:
**07.03.84 Bulletin 84/10**

(45) Publication of the grant of the patent:
**30.12.86 Bulletin 86/52**

(84) Designated Contracting States:
**AT BE CH DE FR GB IT LI NL SE**

(56) References cited:
**EP-A-0 047 005**
**GB-A-2 086 905**

(73) Proprietor: **DAIICHI SEIYAKU CO. LTD.**
**14-10, Nihonbashi 3-chome**
**Chuo-ku Tokyo (JP)**

(72) Inventor: **Hayakawa, Isao Daiichi Seiyaku Research Lab.**
**No. 16-13, Kitakasai 1-chome**
**Edogawa-ku Tokyo (JP)**
Inventor: **Tanaka, Yoshiaki Daiichi Seiyaku Research Lab.**
**No. 16-13, Kitakasai 1-chome**
**Edogawa-ku Tokyo (JP)**

(74) Representative: **Kinzebach, Werner, Dr.**
**Patentanwälte Reitstötter, Kinzebach und Partner Sternwartstrasse 4 Postfach 86 06 49**
**D-8000 München 86 (DE)**

**EP 0 101 829 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

This invention relates to novel tricyclic compounds which are useful as antibacterial agents.

Background of the Invention

European Patent Application (OPI) No. 47005 describes 9 - fluoro - 10 - substituted - 3 - methyl - 7 - oxo - 2,3 - dihydro - 7H - pyrido - (1,2,3 - de) - 1,4 - benzoxazine - 6 - carboxylic acid and West German Patent Application (OPI) No. 2914258 describes 9 - fluoro - 8 - (4 - methyl - 1 - piperazinyl) - 5 - methyl - 6,7 - dihydro - 1 - oxo - 1H,5H - benz(i,j)quinolizine - 2 - carboxylic acid (the term "OPI" as used herein refers to a "published unexamined application").

The present inventors found that introduction of a methylene group ($=CH_2$) into the tricyclic compounds brings the excellent antibacterial activity and completed this invention.

Detailed Description of the Invention

This invention relates to a novel antibacterial agent, and more particularly to tricyclic compounds of formula (I)

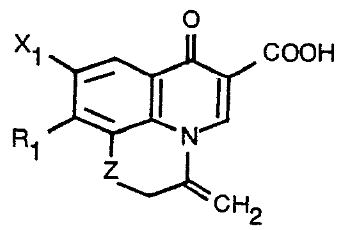

(I)

wherein:

$X_1$ represents a hydrogen atom or a halogen atom,

Z represents an oxygen atom or a methylene group, and

$R_1$ represents an amino group which is part of a 4- to 7-membered cyclic group containing optionally one or more additional N, S or O atom(s) and being optionally substituted with one or more substituents selected from the group consisting of hydroxyl group, amino group, alkyl group, mono- or di-alkylamino group, hydroxyalkyl group and aminoalkyl group

whereby the above-mentioned alkyl moieties have 1 to 6 carbon atoms

and the physiologically acceptable salts thereof.

Examples for the amino group which is part of a 4 to 7-membered cyclic ring containing optionally one or more additional N, S or O atom(s) are:

1-azetidinyl, 1-pyrrolidinyl, 1-piperidinyl, 1-piperazinyl, 4-morpholinyl, 1-homopiperazinyl and the like as well as the substituted groups thereof such as 3-hydroxy-1-pyrrolidinyl, 3-alkylamino-1-pyrrolidinyl, 3-amino-1-pyrrolidinyl and 4-methyl-1-piperazinyl.

The compound of this invention can form an acid addition salt with an inorganic acid such as hydrochloric acid and sulfuric acid or an organic acid such as acidic amino acids, e.g. aspartic acid and glutamic acid, uronic acids, e.g. glucuronic acid and galacturonic acid, sulfonic acids, e.g. methanesulfonic acid, carboxylic acids, e.g. tartaric acid. Moreover, the compounds of this invention can form the corresponding carboxylate with an alkali metal or an alkaline earth metal such as sodium, potassium or calcium.

The compounds of this invention have excellent anti-bacterial activity against Gram-positive and Gram-negative bacteria, particularly, against *Pseudomonas aeruginosa*.

Referring to $X_1$ in the structural formula (I), halogen atom, especially, fluorine atom is preferred and referring to $R_1$, substituted pyrrolidinyl group, especially, 3-amino-1-pyrrolidinyl group and 3-hydroxy-1-pyrrolidinyl group are preferred. A particularly preferred class of compounds is those having formula (I) wherein $X_1$ is a fluorine atom and $R_1$ is a 3-hydroxy-1-pyrrolidinyl group or a 3-amino-1-pyrrolidinyl group.

The process for preparing the compound of formula (I) is illustrated by the following reaction scheme:

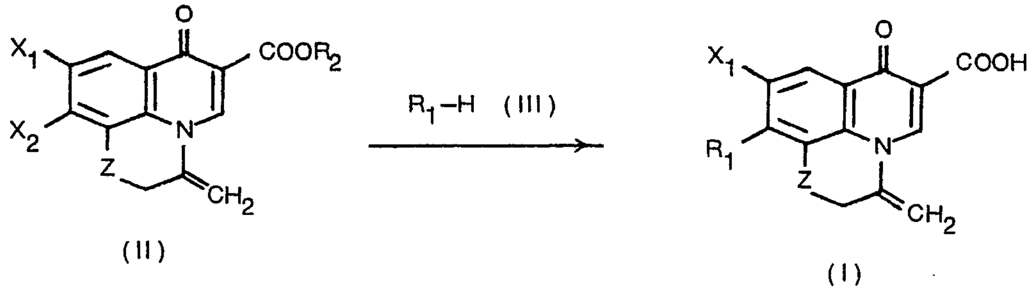

wherein $X_1$, Z and $R_1$ are as defined above, $R_2$ represents a hydrogen atom or an alkyl group and $X_2$ represents a halogen atom.

The reaction of the compound of formula (II) with the compound of formula (III) is usually performed in the absence of solvent or in the presence of a polar solvent such as water, alcohols, dimethylformamide, dimethylacetamide, dimethyl sulfoxide, pyridine and the like for 1 hour to 6 hours at 50°C to 200°C, preferably, at 100°C to 150°C. Alternatively, the above reaction can be performed in the presence of an acid acceptor such as a tertiary amine, e.g. triethylamine and dimethylaniline, an inorganic base, e.g. potassium carbonate and the like, at a molar ratio of 1.0 to 1.2 of the acid acceptor per mole of the compound of formula (II). When the reaction is performed in the presence of acid acceptor, the compound of formula (III) can be preferably employed in 1.0 mole to 1.2 moles per mole of the compound of formula (II) and when the reaction is performed in the absence of the acid acceptor, the compound of formula (III) can be preferably employed in 2 moles to 5 moles per mole of the compound of formula (II).

When the compound of formula (III) is a cyclic amine substituted with an amino group, an aminoalkyl group or a mono-alkylamino group, the amino moiety is protected with a protecting group and the protected compound is allowed to react with the compound of formula (II). Thereafter, the protecting group can be eliminated from the resulting compound to produce the objective compound of formula (I).

The reactions used for eliminating the protecting group include a usual hydrolysis with an acid or a base and a usual catalytic reduction.

The hydrolysis is favorable for eliminating the protecting groups such as tertiary butoxycarbonyl group, ethoxycarbonyl group, acyl group, e.g. acetyl group and trifluoroacetyl group or tosyl group. And the catalytic reduction is favorable for eliminating the protecting groups such as 4-methoxybenzyl group, benzyl group or benzhydryl group.

When the compound of formula (II) wherein $R_2$ is an alkyl group is reacted with the compound of formula (III), the ester moiety of the product can be decomposed by hydrolysis with an acid or a base.

The hydrolysis of ester moiety with a base is usually performed in a solvent such as an aqueous alcohol or a mixture of water and an organic polar solvent, e.g. dimethyl sulfoxide and dimethylformamide, for 15 minutes to 2 hours at room temperature to 100°C. Examples of the base include an inorganic base such as an alkali metal or alkaline earth metal hydroxide or carbonate and the like. The base is usually employed in an amount of 1 mole to 5 moles per mole of the ester compound.

The hydrolysis of ester moiety with an acid is usually performed by heating the ester compound in an inorganic acid such as hydrochloric acid, or a mixture of an inorganic acid and an alcohol such as methanol under reflux for 30 minutes to 5 hours or by heating the ester compound in a mixture of an inorganic acid and an organic acid such as acetic acid for 30 minutes to 5 hours at 100°C to 130°C. The inorganic acid is usually employed in an amount of 2 moles to 10 moles per mole of the ester compound. When the inorganic acid is employed in excess, it can act as a solvent as well.

Apparently from the description with respect to the elimination of protecting group and decomposition of ester moiety, it is possible to complete the above two reactions together by hydrolysis.

When Z in formula (II) represents an oxygen atom, the starting material of the formula (II) can be prepared by the process outlined below:

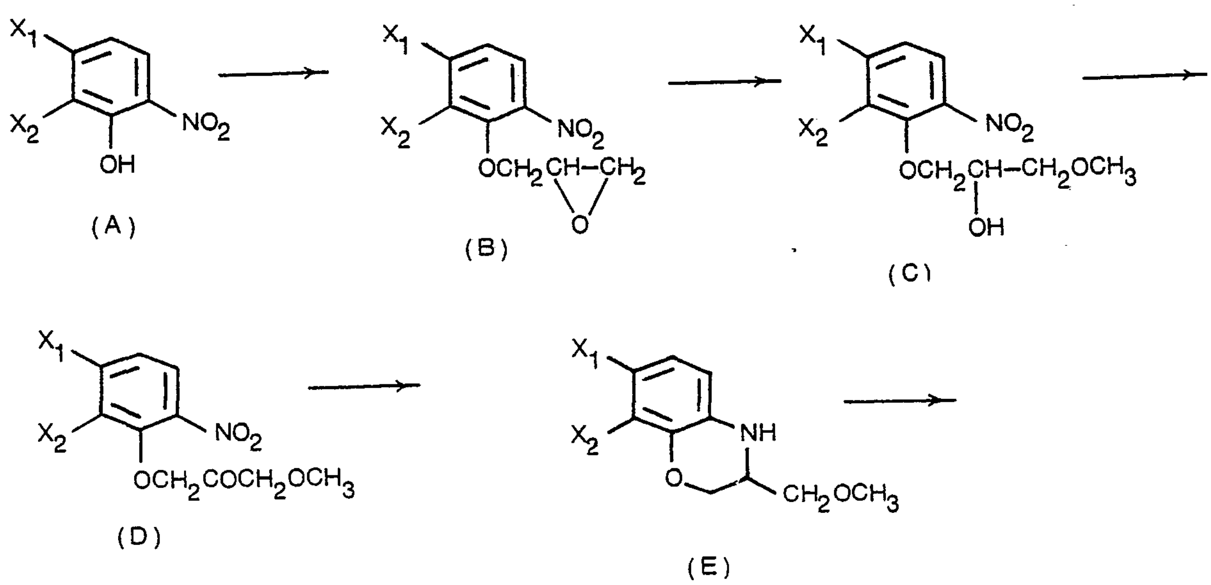

(A) (B) (C) (D) (E)

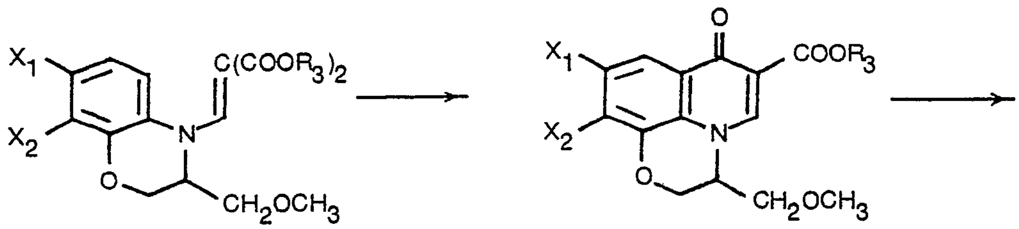

(F) (G)

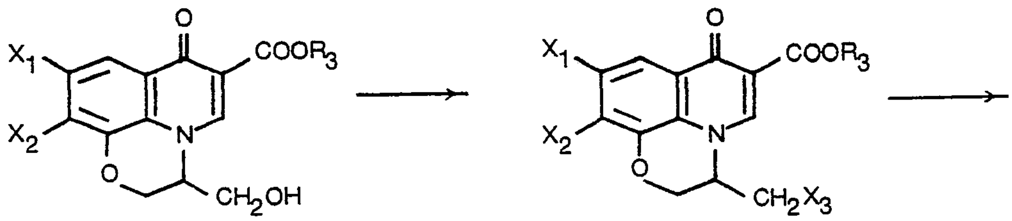

(H) (J)

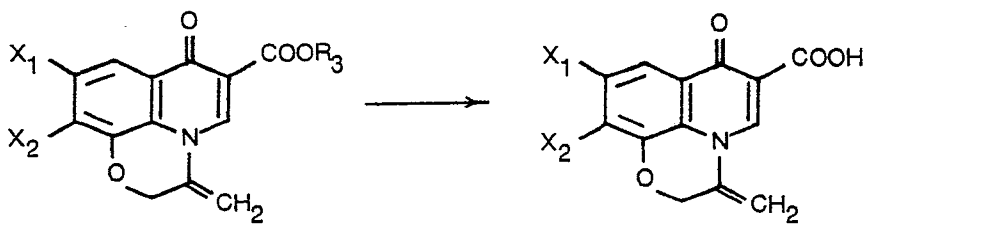

(IIb) (IIa)

wherein $X_1$ and $X_2$ are as defined above, $X_3$ represents a halogen atom and $R_3$ represents an alkyl group.

That is, the compound of formula (A) is allowed to react with an epihalogenohydrin in the presence of an acid acceptor to produce the compund of formula (B) and the product is heated with a catalytic amount of tin dichloride in an alcohol to produce the compound of formula (C). The compound of formula (C) is treated with an oxidizing agent containing anhydrous chromic acid such as the Jones reagent to produce the compound of formula (D) and the product is reduced in the presence of a catalyst such as Raney nickel, palladium black and the like to produce the compound of formula (E). The compound of formula (E) is heated with a dialkyl alkoxymethylenemalonate to produce the compound of formula (F) and the product is heated in a polyphosphoric acid or an ester thereof to product the compound of formula (G). The compound of formula (G) is selectively hydrolyzed to produce the compound of formula (H) by treating with an aluminum halogenide and the product is treated with a halogenating agent such as thionyl chloride to produce the compound of formula (J). The compound of formula (J) is treated with an acid acceptor such as a tertiary amine, specifically, 1,8-diazabicyclo(5,4,0)-7-undecane, to produce the compound of formula (IIb) and then the product is hydrolyzed in a similar manner as described before to produce the compound of formula (IIa).

Also, when Z in formula (II) represents a methylene group, the starting material of formula (II) can be prepared by a somewhat different process which is shown below:

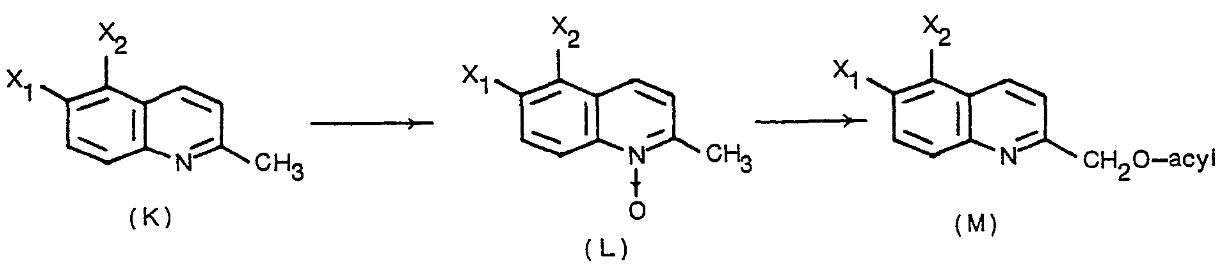

(K) (L) (M)

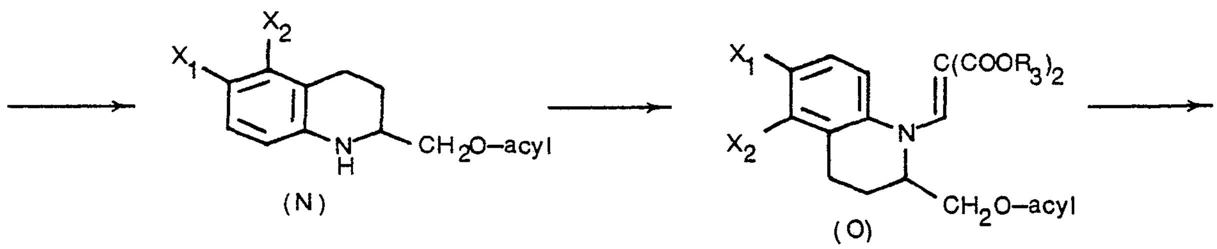

(N) (O)

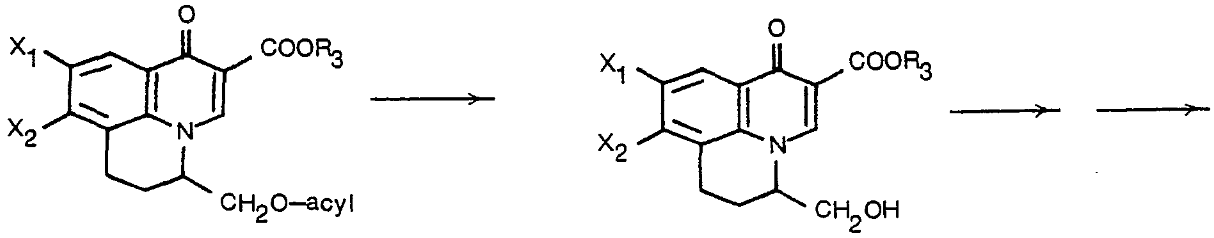

(P) (Q)

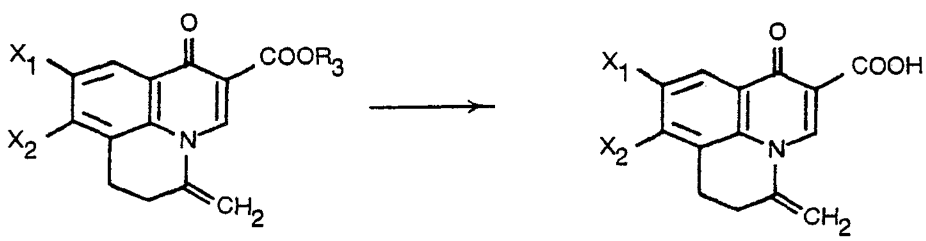

(IId) (IIc)

wherein $X_1$, $X_2$ and $R_3$ are as defined above.

That is, the compound of formula (K) is treated with a peroxydizing agent such as an organic peracid, e.g. 3-chloroperbenzoic acid, or hydrogen peroxide to produce the compound of formula (L) and the product is heated in an acid anhydride such as acetic anhydride to produce the compound of formula (M) and then the product is reduced in the presence of a catalyst such as platinum oxide, palladium black and the like under normal atmospheric pressure or under pressurized condition to produce the compound of formula (N). The compound of formula (N) is heated with a dialkyl alkoxymethylenemalonate to give the compound of formula (O) and the product is heated in a polyphosphate to produce the compound of formula (P) and then the product is selectively hydrolyzed to product the compound of formula (Q). The compound of formula (Q) is treated in the same procedure as described above to produce the compounds of (IId) and (IIc).

The antibacterial activity of the compound of this invention is shown in the following Table 1.

TABLE 1

MIC (mcg/ml)*

| Test Organism | Ia | Ib | Ic | Id |
|---|---|---|---|---|
| E. coli, NIHJ | 0.0125 | 0.025 | 0.0125 | 0.0125 |
| Sh. flexneri, 2a, 5503 | 0.025 | 0.0125 | 0.0125 | 0.0125 |
| Pr. vulgaris, 08602 | 0.025 | 0.025 | 0.05 | 0.025 |
| Pr. mirabilis, IFO—3849 | 0.05 | 0.025 | 0.10 | 0.025 |
| Kleb. pneumoniae, type 1 | 0.19 | 0.05 | 0.10 | 0.05 |
| Ent. cloacae, 03400 | 0.05 | 0.025 | 0.05 | 0.0125 |
| Ser. marcescens, 10104 | 0.10 | 0.05 | 0.10 | 0.05 |
| Ps. aeruginosa, 32104 | 0.78 | 0.10 | 0.31 | 0.05 |
| Ps. aeruginosa, 32233 | 0.78 | 0.19 | 0.78 | 0.10 |
| Ps. aeruginosa, 32234 | 0.78 | 0.10 | 0.39 | 0.05 |
| Ps. aeruginosa, 32121 | 0.19 | 0.05 | 0.10 | 0.025 |
| Ps. aeruginosa, 32122 | 0.78 | 0.10 | 0.39 | 0.10 |
| S. aureus, 209 P | 0.05 | 0.10 | 0.05 | 0.10 |
| S. epidermidis, 56500 | 0.19 | 0.39 | 0.10 | 0.19 |
| Str. pyogenes, G—36 | 0.78 | 0.78 | 0.39 | 0.39 |
| Str. faecalis, ATCC—19433 | 0.39 | 0.78 | 0.39 | 0.39 |
| B. subtilis, ATCC—6633 | ≦0.0063 | 0.05 | ≦0.0063 | 0.025 |

* Determined by the standard method of the Japan Society of Chemotherapy: (Mueller-Hinton Broth medium), $10^6$/ml bacteria were seeded and incubated at 37°C for 18 hours.

Ia: 9-fluoro-10-(3-hydroxy-1-pyrrolidinyl)-3-methylene-7-oxo-2,3-dihydro-7H-pyrido(1,2,3-de)-1,4-benzoxazine-6-carboxylic acid

Ib: 10-(3-amino-1-pyrrolidinyl)-9-fluoro-3-methylene-7-oxo-2,3-dihydro-7H-pyrido(1,2,3-de)-1,4-benzoxazine-6-carboxylic acid

Ic: 9-fluoro-8-(3-hydroxy-1-pyrrolidinyl)-5-methylene-1-oxo-6,7-dihydro-1H,5H-benzo(i,j)quinolizine-2-carboxylic acid

Id: 8-(3-amino-1-pyrrolidinyl)-9-fluoro-5-methylene-1-oxo-6,7-dihydro-1H,5H-benzo(i,j)quinolizine-2-carboxylic acid

As can be seen in the Table 1, the compounds of this invention exhibit a very excellent antibacterial activity, particularly, 3-hydroxy-1-pyrrolidinyl compounds (Ia and Ic) exhibit a very strong antibacterial activity against Gram-positive bacteria and 3-amino-1-pyrrolidinyl compounds (Ib and Id) exhibit a very strong antibacterial activity against *Pseudomonas aeruginosa*. The excellency is clearly comprehesible in comparing the Table 1 with the Tables in the specifications of EP Application (OPI) No. 47005 and DE Application (OPI) No. 2914258.

With respect to the toxicity of the compounds of this invention, the acute toxicity ($LD_{50}$) of the compound (Ia, sodium salt) is 333 mg/kg and that of the compound (Ib, methansulfonic acid addition salt) is more than 320 mg/kg and that of the compound (Id, hydrochloric acid addition salt) is 252 mg/kg as determined in mice (i.v.).

Production of Starting Material

Referential Example 1

7.0 g of 2,3-difluoro-6-nitrophenol, 7.0 g of epichlorohydrin, 15 g of potassium carbonate and 600 mg of potassium iodide were added to 150 ml of dimethylformamide and the mixture was stirred for 20 hours at 85—90°C (bath temperature). The insoluble material was removed by filtration and the filtrate was concentrated to dryness in vacuo and then the residue was distributed between chloroform and water. The chloroform layer was washed with water and dried over sodium sulfate. The solvent was distilled off and the residue was purified by silica gel column chromatography to give 6.1 g of 2,3-difluoro-6-nitrophenyl oxyranylmethyl ether as a light yellow oil.

15.0 g of the above product and 0.3ml of tin dichloride were added to 60 ml of anhydrous methanol and the resulting mixture was refluxed for 2 hours. The solvent was distilled off and the residue was distributed between chloroform and water. 16.1 g of 1-(2,3-difluoro-6-nitrophenoxy)-3-methoxy-2-propanol was obtained as an oil from chloroform layer.

15 g of the above product was dissolved in 150 ml of acetone and 50 ml of the Jones reagent prepared from 32 g of anhydrous chromic acid, 64 ml of water and 16 ml of concentrated sulfuric acid was added dropwise to the mixture under stirring and cooling in an ice bath. The resulting mixture was stirred for 30 minutes at the same temperature and for additional 2 hours at room temperature. The insoluble material was collected by filtration and washed with acetone and chloroform. The washings were combined with the filtrate and the mixture was concentrated to dryness and then the residue was distributed between chloroform and water. The chloroform layer was washed with water and dried over sodium sulfate.

The solvent was distilled off and the residue was purified by silica gel column chromatography with chloroform eluent to give 10.6 g of 1-(2,3-difluoro-6-nitrophenoxy)-3-methoxy-2-propanone with mp 39—42°C.

Analysis for $C_{10}H_9F_2NO_5$

| | | | |
|---|---|---|---|
| Calculated | C 45.99, | H 3.47, | N 5.36 |
| Found | C 45.79, | H 3.26, | N 5.29 |

9.5 g of the above product was dissolved in 100 ml of ethanol. After addition of 10 ml of Raney nickel, it was catalytically reduced under normal atmospheric pressure and the catalyst was removed by filtration. The solvent was distilled off in vacuo and the residue was purified by silica gel column chromatography to give 3.9 g of an oily product. 3.5 g of diethyl ethoxymethylenemalonate was added to 3.0 g of the above product and the mixture was heated for 2 hours at 105—115°C (bath temperature). The reaction mixture was purified by silica gel column chromatography to give 4.1 g of diethyl(7,8-difluoro-3-methoxymethyl-2,3-dihydro-4H-1,4-benzoxazin-4-yl)methylenemalonate with mp 81°C.

Analysis for $C_{18}H_{21}F_2NO_6$

| | | | |
|---|---|---|---|
| Calculated | C 56.10, | H 5.49, | N 3.64 |
| Found | C 56.25, | H 5.47, | N 3.74 |

3.0 g of the above product was added to 20 g of polyphosphate (prepared from phosphoric anhydride and ethanol) and the mixture was heated for 1.5 hours at 120—125°C (bath temperature). Ice-cooled water was added to the reaction mixture and the precipitate formed was extracted with chloroform. The extract was washed with water and dried over sodium sulfate. The solvent was distilled off and the residue was purified by silica gel column chromatography with chloroform eluent and then recrystallized from a mixture of dichloromethane and diisopropyl ether to give 1.7 g of ethyl 9,10-difluoro-3-methoxymethyl-7-oxo-2,3-dihydro-7H-pyrido(1,2,3-de)-1,4-benzoxazine-6-carboxylate with mp 238°C as fine needles.

Analysis for $C_{16}H_{15}F_2NO_5$

| | | | |
|---|---|---|---|
| Calculated | C 56.64, | H 4.46, | N 4.13 |
| Found | C 56.51, | H 4.44, | N 4.02 |

1.7 g of the above product was added to 100 ml of dichloromethane and a mixture of 6.0 g aluminum bromide and 10 ml of ethanethiol was added dropwise to the mixture under cooling in an ice bath. The temperature of the resulting mixture was raised to room temperature and the mixture was stirred for 3 hours at the same temperature. The solvent was distilled off and ice-cooled water was added to the residue. The precipitate formed was collected by filtration and recrystallized from a mixture of chloroform and ethanol to give 1.1 g of ethyl 9,10-difluoro-3-hydroxymethyl-7-oxo-2,3-dihydro-7H-pyrido(1,2,3-de)-1,4-benzoxazine-6-carboxylate with mp 268—270°C as fine needles.

Analysis for $C_{15}H_{13}F_2NO_5$

| | | | |
|---|---|---|---|
| Calculated | C 55.39, | H 4.03, | N 4.31 |
| Found | C 55.66, | H 4.23, | N 4.29 |

400 mg of the above product was dissolved in 30 ml of chloroform and 3 ml of thionyl chloride was added to the mixture. The resulting mixture was refluxed for 4 hours. The reaction mixture was concentrated to dryness in vacuo and the residue was dissolved in chloroform. The mixture was washed with water, an aqueous solution of sodium bicarbonate and water and then dried over sodium sulfate. The solvent was distilled off and the residue was recrystallized from a mixture of chloroform and ethanol to give 220 mg of ethyl 3-chloromethyl-9,10-difluoro-7-oxo-2,3-dihydro-7H-pyrido(1,2,3-de)-1,4-benzoxazine-6-carboxylate with mp 250—251°C as fine needles.

Analysis for $C_{15}H_{12}ClF_2NO_4$

| | | | |
|---|---|---|---|
| Calculated | C 52.42, | H 3.52, | N 4.08 |
| Found | C 52.26, | H 3.45, | N 4.10 |

200 mg of the above product was suspended in 30 ml of anhydrous benzene and 230 mg of 1,8-diazabicyclo(5,4,0)-7-undecene was added to the mixture and then the resulting mixture was refluxed for one hour. Chloroform was added to the reaction mixture and the mixture was washed with water and dried over sodium sulfate. The solvent was distilled off and the residue was purified by silica gel column chromatography and recrystallized from a mixture of dichloromethane and diisopropyl ether to give 120 mg of ethyl 9,10-difluoro-3-methylene-7-oxo-2,3-dihydro-7H-pyrido(1,2,3-de)-1,4-benzoxazine-6-carboxylate with mp 258—263°C.

NMR (DMSO-$d_6$, δ ppm)
5.47, 5.89 (each 1h, d, J=2.5Hz, $C_3=CH_2$)

Analysis for $C_{15}H_{11}F_2NO_4$

| | | | |
|---|---|---|---|
| Calculated | C 58.64, | H 3.61, | N 4.56 |
| Found | C 58.22, | H 3.33, | N 4.47 |

Referential Example 2

1.0 g of 5,6-difluoro-2-methylquinoline was dissolved in 30 ml of chloroform and then a mixture of 20 ml of chloroform and 1.0 g of 3-chloroperbenzoic acid was added dropwise thereto under cooling in an ice bath. The resulting mixture was stirred for 2 hours at 0°C and for additional one hour at room temperature. The reaction mixture was washed twice with a saturated aqueous solution of sodium bicarbonate and washed with a saturated aqueous solution of sodium chloride. After drying, the solvent was distilled off in vacuo and the residue was purified by silica gel column chromatography to give 830 mg of 5,6-difluoro-2-methylquinoline-1-oxode. The product was recrystallized from a mixture of benzene and hexane to give a white powder with mp 125—127°C.

Analysis for $C_{10}H_7F_2NO$

| | | | |
|---|---|---|---|
| Calculated | C 61.54, | H 3.62, | N 7.18 |
| Found | C 61.60, | H 3.70, | N 7.12 |

550 mg of the above product was added to 20 ml of acetic anhydride and the mixture was allowed to react for 15 minutes at 140°C. The solvent was distilled off in vacuo and the residue was purifed by silica gel column chromatography to give 430 mg of 2-acetoxymethyl-5,6-difluoroquinoline.

11.2 g of the above product was reduced in the presence of platinum oxide catalyst and methanol under high pressure (4.5 kg/cm$^2$) to give 4.45 g of 2-acetoxymethyl-5,6-difluoro-1,2,3,4-tetrahydroquinoline.

5.6 g of the above product was allowed to react with diethyl ethoxymethylenemalonate for 8 hours at 140°C. To 3.0 g of the product obtained 7.5 g of polyphosphate (prepared from phosphoric anhydride and ethanol) was added and the mixture was allowed to react for 40 minutes at 140°C to give 610 mg of a product. The product was recrystallized from a mixture of ethanol and chloroform to give ethyl 5-acetoxymethyl-8,9-difluoro-1-oxo-6,7-dihydro-1H,5H-benzo(i,j)quinolizine-2-carboxylate with mp 201—203°C as colorless needles.

Analysis for $C_{18}H_{17}F_2NO_5$

| | | | |
|---|---|---|---|
| Calculated | C 59.18, | H 4.69, | N 3.83 |
| Found | C 58.93, | H 4.73, | N 3.91 |

580 mg of the above product was suspended in 20 ml of ethanol and then 1 ml of a 2 normal aqueous solution of sodium hydroxide and 30 ml of chloroform were added thereto. The resulting mixture was stirred for 20 minutes at room temperature. The volume of the reaction mixture was reduced to a half volume by concentration in vacuo and 50 ml of water was added thereto. The precipitate formed was collected by filtration to give 360 mg of ethyl 8,9-difluoro-5-hydroxymethyl-1-oxo-6,7-dihydro-1H,5H-benzo(i,j)quinolizine-2-carboxylate with mp 256—258°C.

Analysis for $C_{16}H_{15}F_2NO_4.1/4H_2O$

| | | | |
|---|---|---|---|
| Calculated | C 58.63, | H 4.77, | N 4.27 |
| Found | C 58.59, | H 4.57, | N 4.38 |

352 mg of the above product was suspended in 30 ml of chloroform and 3 ml of thionyl chloride was added thereto. The resulting mixture was refluxed for 30 minutes and the solvent was distilled off in vacuo. The residue was dissolved in chloroform and the mixture was washed with an aqueous solution of sodium bicarbonate and water and then dried over sodium sulfate. The solvent was distilled off and the residue was recrystallized from ethanol to give 342 mg of ethyl 5-chloromethyl-8,9-difluoro-1-oxo-6,7-dihydro-1H,5H-benzo(i,j)quinolizine-2-carboxylate with mp 215—218°C as light yellow needles.

Analysis for $C_{16}H_{14}ClF_2NO_3$

| | | | |
|---|---|---|---|
| Calculated | C 56.23, | H 4.13, | N 4.10 |
| Found | C 56.06, | H 4.14, | N 4.04 |

350 mg of the above product was suspended in 50 ml of dehydrated benzene and 350 mg of 1,8-diazabicyclo(5,4,0)-7-undecene (hereinafter abbreviated as DBU) was added thereto. The resulting mixture was refluxed for 2 hours. 300 mg of DBU was added to the reaction mixture and the resulting mixture was refluxed for 3 days. The solvent was distilled off in vacuo. The residue was purified by silica gel column chromatography and then recrystallized from ethanol to give 253 mg of ethyl 8,9-difluoro-5-methylene-1-oxo-6,7-dihydro-1H,5H-benzo(i,j)quinolizine-2-carboxylate with mp 272—274°C as colorless needles.

Analysis for $C_{16}H_{13}F_2NO_3$

| | | | |
|---|---|---|---|
| Calculated | C 62.95, | H 4.29, | N 4.59 |
| Found | C 62.91, | H 4.30, | N 4.59 |

240 mg of the above product was added to a mixture of 1 ml of a normal aqueous solution of sodium hydroxide, 20 ml of water and 30 ml of ethanol and then the resulting mixture was allowed to react for 30 minutes at 40—50°C. Ethanol was distilled off and 40 ml of a 10% aqueous solution of citric acid was added thereto. The precipitate formed was collected by filtration and recrystallized from ethanol to give 180 mg of 8,9-difluoro-5-methylene-1-oxo-6,7-dihydro-1H,5H-benzo(i,j)quinolizine-2-carboxylic acid with mp 220—232°C.

NMR (DMSO-$d_6$, δ ppm)
5.30, 5.61 (each 1H, J=3.0Hz, $C_5=CH_2$)

Analysis for $C_{14}H_9F_2NO_3$

| | | | |
|---|---|---|---|
| Calculated | C 60.66, | H 3.27, | N 5.05 |
| Found | C 60.69, | H 3.52, | N 5.02 |

## Example 1

100 mg of ethyl 9,10-difluoro-3-methylene-7-oxo-2,3-dihydro-7H-pyrido(1,2,3-de)-1,4-benzoxazine-6-carboxylate was dissolved in 3 ml of dimethyl sulfoxide and 100 mg of N-methylpiperazine was added to the mixture and then the resulting mixture was stirred for 6 hours at 120—130°C (bath temperature). The solvent was distilled off in vacuo and the residue was purified by silica gel column chromatography to give 100 mg of a powder. The powder was suspended in 10 ml of ethanol and 1 ml of 3% aqueous solution of sodium hydroxide was added to the mixture. The resulting mixture was stirred for 30 minutes at 40—50°C. The reaction mixture was concentrated to dryness in vacuo and water was added to the residue. The mixture was acidified by addition of diluted hydrochloric acid and was made basic with sodium bicarbonate and then extracted with chloroform. The extract was dried over sodium sulfate and the solvent was distilled off. The residue was purified by silica gel column chromatography and recrystallized from ethanol to give 25 mg of 9-fluoro-10-(4-methyl-1-piperazinyl)-3-methylene-7-oxo-2,3-dihydro-7H-pyrido-(1,2,3-de),-1,4-benzoxazine-6-carboxylic acid with mp 200—201°C as fine needles.

NMR ($CDCl_3$, δ ppm)
4.83 (2H, S, $C_2$—$H_2$)
5.26, 5.61 (each 1H, d, J=3.0 Hz, $C_3=CH_2$)
8.83 (1H, s, $C_5$—H)
7.63 (1H, d, J=12Hz, $C_8$—H)

Analysis for $C_{18}H_{18}FN_3O_4.1/2H_2O$

| | | | |
|---|---|---|---|
| Calculated | C 58.69, | H 5.20, | N 11.41 |
| Found | C 58.98, | H 4.97, | N 11.35 |

## Example 2

900 mg of ethyl 9,10-difluoro-3-methylene-7-oxo-2,3-dihydro-7H-pyrido(1,2,3-de)-1,4-benzoxazine-6-carboxylate was suspended in 20 ml of ethanol and 5 ml of an aqueous solution containing 500 mg of potassium hydroxide was added thereto. The resulting mixture was allowed to react for 3 hours at 50—60°C (bath temperature). The solvent was distilled off and 10 ml of water was added to the residue and the mixture was neutralized by addition of hydrochloric acid.

The precipitate formed was collected by filtration and washed with water and then dried to give 810 mg of 9,10-difluoro-3-methylene-7-oxo-2,3-dihydro-7H-pyrido-(1,2,3-de)-1,4-benzoxazine-6-carboxylic acid as a white powder. The powder was recrystallized from ethanol to give fine needles with mp 273—276°C (decomposition).

NMR (DMSO-$d_6$, δ ppm)
5.14 (2H, s, $C_2$—$H_2$)
5.66, 6.13 (each 1H, d, J=2Hz, $C_3$=$CH_2$)
Analysis for $C_{13}H_7F_2NO_4$

| | | | |
|---|---|---|---|
| Calculated | C 55.92, | H 2.53, | N 5.02 |
| Found | C 55.69, | H 2.70, | N 4.93 |

100 mg of the above product and 200 mg of 3-hydroxypyrrolidine were added to 3 ml of dimethyl sulfoxide and the mixture was stirred for 6 hours at 120—130°C (bath temperature). The solvent was distilled off in vacuo and the residue was washed twice with water and with a mixture of diethyl ether and ethanol (4:1 by volume) and then dried to give a yellow powder. The powder was recrystallized from ethanol to give 72 mg of 9-fluoro-10-(3-hydroxy-1-pyrrolidinyl)-3-methylene-7-oxo-2,3-dihydro-7H-pyrido-(1,2,3-de)-1,4-benzoxazine-6-carboxylic acid as yellow needles with mp 288—289°C (decomposition).

Analysis for $C_{17}H_{15}FN_2O_5$

| | | | |
|---|---|---|---|
| Calculated | C 58.96, | H 4.37, | N 8.09 |
| Found | C 59.07, | H 4.63, | N 8.01 |

Example 3

130 mg of 9,10-difluoro-3-methylene-7-oxo-2,3-dihydro-7H-pyrido(1,2,3-de)-1,4-benzoxazine-6-carboxylic acid and 300 mg of 3-tertiary-butoxycarbonylaminopyrrolidine were added to 3 ml of dimethyl sulfoxide and the mixture was allowed to react for 3 hours at 100—110°C (bath temperature). The solvent was distilled off in vacuo. The residue was washed with diethyl ether and purified by silica gel column chromatography and then recrystallized from benzene to give 125 mg of 10-(3-tertiary-butoxycarbonylamino-1-pyrrolidinyl)-9-fluoro-3-methylene-7-oxo-2,3-dihydro-7H-pyrido(1,2,3-de)-1,4-benzoxazine-6-carboxylic acid with mp 219—220°C as luster yellow crystals.

80 mg of the above product was dissolved in 2 ml of trifluoroacetic acid and 1 ml of anisole was added to the mixture and then the resulting mixture was allowed to stand overnight at room temperature. The reaction mixture was concentrated to dryness in vacuo and an aqueous solution of sodium bicarbonate was added to the residue until the mixture became weakly basic. The precipitate formed was collected by filtration and washed with water. The precipitate was dried and recrystallized from a large amount of a mixture of chloroform and ethanol to give 45 mg of 10-(3-amino-1-pyrrolidinyl)-9-fluoro-3-methylene-7-oxo-2,3-dihydro-7H-pyrido(1,2,3-de)-1,4-benzoxazine-6-carboxylic acid as light yellow needles with mp 260—265°C (decomposition).

NMR (DMSO-$d_6$, δ ppm)
4.83 (2H, s, $C_2$—$H_2$)
5,16 5.55 (each 1H, $C_3$=$CH_2$)
Analysis for $C_{17}H_{16}FN_3O_4.1/2H_2O$

| | | | |
|---|---|---|---|
| Calculated | C 57.63, | H 4.83, | N 11.86 |
| Found | C 58.03, | H 4.78, | N 11.90 |

Example 4

600 mg of 10-(3-amino-1-pyrrolidinyl)-9-fluoro-3-methylene-7-oxo-2,3-dihydro-7H-pyrido(1,2,3-de)-1,4-benzoxazine-6-carboxylic acid was suspended in a mixture of 20 ml of water and 20 ml of methanol and then 2.2 ml of 1 normal hydrochloric acid was added thereto to give a yellow solution. Insoluble material was removed by filtration and the filtrate was concentrated to dryness in vacuo. 30 ml of ethanol and 20 ml of diethyl ether were added to the residue in a slurry form. The precipitate formed was collected by filtration and washed with diethyl ether and then dried for 30 hours at 70—80°C under reduced pressure (1 mm Hg) to give 600 mg of the corresponding hydrochloric acid salt as yellow fine needles.

The salt melted at 270—285°C and gradually decomposed at that temperature.

Analysis for $C_{17}H_{16}FN_3O_4.HCl.1/4H_2O$

| | | | |
|---|---|---|---|
| Calculated | C 52.86, | H 4.50, | N 10.88 |
| Found | C 52.75, | H 4.51, | N 10.87 |

Example 5

220 mg of 10-(3-amino-1-pyrrolidinyl)-9-fluoro-3-methylene-7-oxo-2,3-dihydro-7H-pyrido(1,2,3-de)-1,4-benzoxazine-6-carboxylic acid was suspended in 10 ml of ethanol and 10 ml of an ethanol solution containing 100 mg of methanesulfonic acid was added dropwise thereto at room temperature uder stirring. The resulting mixture was stirred for 15 minutes at 50—60°C and then the reaction mixture was concentrated to dryness at room temperature. The yellow residue was washed with anhydrous ethanol and then dried for 40 hours at 60°C under reduced pressure (1 mm Hg) to give 250 mg of the corresponding methanesulfonic acid salt as a yellow powder with mp 263—265°C (decomposition).

Analysis for $C_{17}H_{16}FN_3O_4.CH_3SO_3H$

| | | | |
|---|---|---|---|
| Calculated | C 48.98, | H 4.57, | N 9.52 |
| Found | C 49.20, | H 4.60, | N 9.55 |

Example 6

150 mg of 8,9-difluoro-5-methylene-1-oxo-6,7-dihydro-1H,5H-benzo(i,j)quinolizine-2-carboxylic acid was suspended in 3 ml of dimethyl sulfoxide and 150 mg of 3-tertiary-butoxycarbonylaminopyrrolidine was added thereto. The resulting mixture was allowed to react for 3.5 hours at 120°C. The solvent was distilled off and the residue was purified by preparative thin layer chromatography on silica gel to give a crude product.

6 ml of trifluoroacetic acid and 250 mg of anisole were added to the above product and the resulting mixture was stirred for one hour at room temperature. The solvent was distilled off and then 4 ml of a saturated aqueous solution of sodium bicarbonate and 10 ml of methanol were added to the residue to dissolve it. The solvent was distilled off. The residue was purified by column chromatography on HP 20 (Diaion HP—20, a product of Mitsubishi Chemical Industries Ltd.) and purified by high performance liquid chromatography on μ Bondapac $C_{18}$ (a product of Waters Associates) using a mixture of water and acetonitrile (2:1 by volume) for elution and then recrystallized from ethanol to give 43 mg of 8-(3-amino-1-pyrrolidinyl)-9-fluoro-5-methylene-1-oxo-6,7-dihydro-1H,5H-benzo(i,j)quinolizine-2-carboxylic acid as microcrystals with decomposing point 152—170°C.

NMR (DMSO-$d_6$, δ ppm)
1.70, 2.10 (each 1H, m, $C_4$—H of pyrrolidine)
2.77, 3.02 (each 2H, t, $C_6$— and $C_7$—H)
3.2—3.6 (5H, m, pyrrolidine)
5.26, 5.28 (each 1H, d, J=2Hz, $C_5=CH_2$)
7.84 (1H, d, J≦14Hz, $C_{10}$—H)
8.80 (1H, s, $C_3$—H)

Analysis for $C_{18}H_{18}FN_3O_3.3/4H_2O$

| | | | |
|---|---|---|---|
| Calculated | C 60.58, | H 5.51, | N 11.77 |
| Found | C 60.57, | H 5.61, | N 11.51 |

Example 7

4 ml of dimethyl sulfoxide and 100 mg of 3-hydroxypyrrolidine were added to 100 mg of 8,9-difluoro-5-methylene-1-oxo-6,7-dihydro-1H,5H-benzo(i,j)quinolizine-2-carboxylic acid and the resulting mixture was allowed to react for 2 hours at 120°C. The solvent was distilled off. The residue was purified by silica gel column chromatography and purified by preparative thin layer chromatography on silica gel and then recrystallized from a mixture of ethanol and chloroform to give 28 mg of 9-fluoro-8-(3-hydroxy-1-pyrrolidinyl)-5-methylene-1-oxo-6,7-dihydro-1H,5H-benzo(i,j)quinolizine-2-carboxylic acid as microcrystals with mp 190—196°C.

NMR ($CDCl_3$, δ ppm)
5.16, 5.41 (each 1H, d, J=2Hz, $C_5=CH_2$)
8.04 (1H, d, J=14Hz, $C_{10}$—H)
8.97 (1H, s, $C_3$—H)

Analysis for $C_{18}H_{17}FN_2O_4.1/4H_2O$

| | | | |
|---|---|---|---|
| Calculated | C 61.97, | H 5.06, | N 8.02 |
| Found | C 62.24, | H 4.80, | N 8.19 |

Example 8

400 mg of N-methylpiperazine and 6 ml of dimethyl sulfoxide were added to 400 mg of 8,9-difluoro-5-methylene-6,7-dihydro-1-oxo-1H,5H-benzo(i,j)quinolizine-2-carboxylic acid and the resulting mixture was heated for 2.5 hours at 100—110°C (bath temperature). The solvent was distilled off in vacuo and water was added to the residue. The mixture was extracted with chloroform and the extract was dried. The solvent was distilled off and the residue was purified by silica gel column chromatography and then recrystallized

from ethanol to give 85 mg of 8-(4-methyl-1-piperazinyl)-9-fluoro-5-methylene-6,7-dihydro-1-oxo-1H,5H-benzo(i,j)quinolizine-2-carboxylic acid as microcrystals with 282—285°C (decomposition).

Analysis for $C_{19}H_{20}N_3O_3F.1/4H_2O$

| | | | |
|---|---|---|---|
| Calculated | C 63.06, | H 5.71, | N 11.61 |
| Found | C 63.16, | H 5.64, | N 11.50 |

Example 9

1.5 ml of 2 normal hydrochloric acid and 20 ml of methanol were added to 600 mg of 8-(3-amino-1-pyrrolidinyl)-9-fluoro-5-methylene-6,7-dihydro-1-oxo-1H,5H-benzo(i,j)quinolizine-2-carboxylic acid to dissolve it. The solvent was distilled off at a temperature slightly lower than room temperature. Ethanol was added to the residue and the mixture was concentrated to dryness in vacuo and methanol was added to the residue. The precipitate formed was collected by filtration and washed with ethanol and diethyl ether and then dried. The precipitate was recrystallized from methanol to give 245 mg of the corresponding hydrochloric acid salt. The salt melted at 182—187°C and gradually decomposed at that temperature.

Analysis for $C_{18}H_{18}FN_3O_4.HCl.H_2O$

| | | | |
|---|---|---|---|
| Calculated | C 53.34, | H 5.32, | N 10.56 |
| Found | C 54.09, | H 5.32, | N 10.30 |

**Claims for the Contracting States: BE CH DE FR GB IT LI NL SE**

1. Compounds of the general formula (I):

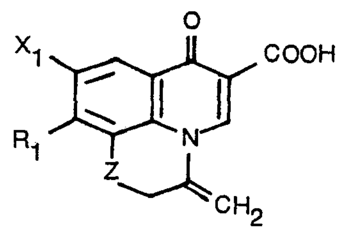

(I)

wherein:

$X_1$ represents a hydrogen atom or a halogen atom,

Z represents an oxygen atom or a methylene group, and

$R_1$ represents an amino group whcih is part of a 4- to 7-membered cyclic group containing optionally one or more additional N, S or O atom(s) and being optionally substituted with one or more substituents selected from the group consisting of hydroxyl group, amino group, alkyl group, mono- or di-alkylamino group, hydroxyalkyl group and aminoalkyl group

whereby the above-mentioned alkyl moieties have 1 to 6 carbon atoms

and the physiologically acceptable salts thereof.

2. Compounds as claimed in Claim 1, wherein $X_1$ is a fluorine atom.

3. Compounds as claimed in Claim 1, wherein $X_1$ is a fluorine atom and $R_1$ is a substituted pyrrolidinyl group.

4. Compound as claimed in Claim 1, wherein Z is an oxygen atom, $X_1$ is a fluorine atom and $R_1$ is a 3-hydroxy-1-pyrrolidinyl group.

5. Compound as claimed in Claim 1, wherein Z is an oxygen atom, $X_1$ is a fluorine atom and $R_1$ is a 3-amino-1-pyrrolidinyl group.

6. Compound as claimed in Claim 1, wherein Z is a methylene group, $X_1$ is a fluorine atom and $R_1$ is a 3-hydroxy-1-pyrrolidinyl group.

7. Compound as claimed in Claim 1, wherein Z is a methylene group, $X_1$ is a fluorine atom and $R_1$ is a 3-amino-1-pyrrolidinyl group.

8. Compound as claimed in Claim 1, wherein Z is an oxygen atom, $X_1$ is a fluorine atom and $R_1$ is a 4-methyl-1-piperazinyl-group.

9. Process for preparing the compounds of the general formula (I) as claimed in claims 1 to 8, characteized in that a compound of the general formula (II):

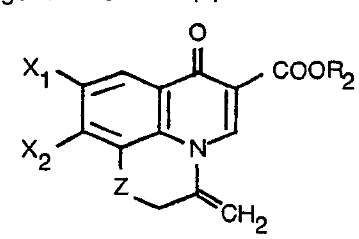

(II)

wherein

$X_1$ and Z are as defined above,

$X_2$ represents a halogen atom, and

$R_2$ represents a hydrogen atom or an alkyl group, is reacted with a cyclic amine of the formula $R_3$—H, wherein

$R_3$ represents an amino group which is part of a 4- to 7-membered cyclic ring containing optionally one or more additional N, S or O atom(s) and being optionally substituted with one or more substituents selected from the group consisting of hydroxyl group, alkyl group, hydroxyalkyl group, dialkylamino group, protected amino group, protected mono-alkylamino group and protected aminoalkyl group,

whereby the alkyl moieties mentioned above have 1 to 6 carbon atoms,

then, optionally, the protecting group is eliminated and the ester moiety is hydrolyzed, and, if desired, the obtained compound is converted to a physiologically acceptable salt thereof.

10. Pharmaceutical composition containing at least one of the compounds as claimed in claims 1 to 8 and a pharmaceutically acceptable carrier.

**Claim for the Contracting State: AT**

A process for preparing the compounds of the general formula I:

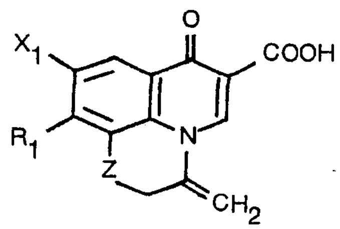

(I)

wherein:

$X_1$ represents a hydrogen atom or a halogen atom,

Z represents an oxygen atom or a methylene group, and

$R_1$ represents an amino group whcih is part of a 4- to 7-membered cyclic group containing optionally one or more additional N, S or O atom(s) and being optionally substituted with one or more substituents selected from the group consisting of hydroxyl group, amino group, alkyl group, mono- or di-alkylamino group, hydroxyalkyl group and aminoalkyl group

whereby the above-mentioned alkyl moieties have 1 to 6 carbon atoms

and the physiologically acceptable salts thereof, characterized in that a compound of the general formula II:

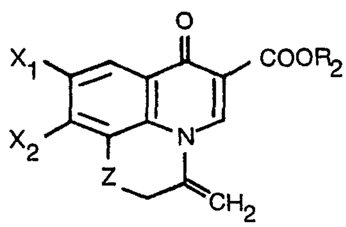

(II)

wherein

$X_1$ and Z are as defined above,

$X_2$ represents a halogen atom, and

$R_2$ represents a hydrogen atom or an alkyl group, is reacted with a cyclic amine of the formula $R_3$—H, wherein

$R_3$ represents an amino group which is part of a 4- to 7-membered cyclic ring containing optionally one or more additional N, S or O atom(s) and being optionally substituted with one or more substituents selected from the group consisting of hydroxyl group, alkyl group, hydroxyalkyl group, dialkylamino group, protected amino group, protected mono-alkylamino group and protected aminoalkyl group,

whereby the alkyl moieties mentioned above have 1 to 6 carbon atoms,

then, optionally, the protecting group is eliminated and the ester moiety is hydrolyzed, and, if desired, the obtained compound is converted to a physiologically acceptable salt thereof.

**Patentansprüche für die Vertragstaaten BE, CH, DE FR, GB, IT, LI, NL, SE**

1. Verbindungen der allgemeinen Formel (I):

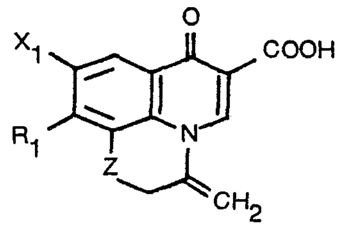

(I)

worin

$X_1$ ein Wasserstoffatom oder ein Halogenatom bedeutet,

Z ein Sauerstoffatom oder eine Methylengruppe bedeutet, und

$R_1$ eine Aminogruppe bedeutet, die Teil einer 4-7-gliedrigen cyclischen Gruppe darstellt, welche gewünschtenfalls ein zusätzliches oder mehrere zusätzliche N-, S- oder O-Atom(e) enthält und gewünschtenfalls substituiert ist durch einen oder mehrere Substituenten, ausgewählt aus der Gruppe bestehend aus einer Hydroxygruppe, Aminogruppe, Alkylgruppe, Mono- oder Dialkylaminogruppe, Hydroxyalkylgruppe und Aminoalkylgruppe,

wobei die oben genannten Alkyleinheiten 1 bis 6 Kohlenstoffatome aufweisen,

und die physiologisch verträglichen Salze davon.

2. Verbindungen nach Anspruch 1, worin $X_1$ ein Fluoratom bedeutet.

3. Verbindungen nach Anspruch 1, worin $X_1$ ein Fluoratom und $R_1$ eine substituierte Pyrrolidinylgruppe bedeuten.

4. Verbindung nach Anspruch 1, worin Z ein Sauerstoffatom, $X_1$ ein Fluoratom und $R_1$ eine 3-Hydroxy-1-pyrrolidinylgruppe bedeuten.

5. Verbindung nach Anspruch 1, worin Z ein Sauerstoffatom, $X_1$ ein Fluoratom und $R_1$ eine 3-Amino-1-pyrrolidinylgruppe bedeuten.

6. Verbindung nach Anspruch 1, worin Z eine Methylengruppe $X_1$ ein Fluoratom und $R_1$ eine 3-Hydroxy-1-pyrrolidinylgruppe bedeuten.

7. Verbindung nach Anspruch 1, worin Z eine Methylengruppe, $X_1$ ein Fluoratom and $R_1$ eine 3-Amino-1-pyrrolidinylgruppe bedeuten.

8. Verbindung nach Anspruch 1, worin Z ein Sauerstoffatom, $X_1$ ein Fluoratom und $R_1$ eine 4-Methyl-1-piperazinylgruppe bedeuten.

9. Verfahren zur Herstellung der Verbindungen der allgemeinen Formel (I) gemäß den Ansprüchen 1 bis 8, dadurch gekennzeichnet, daß man eine Verbindung der allgemeinen Formel (II)

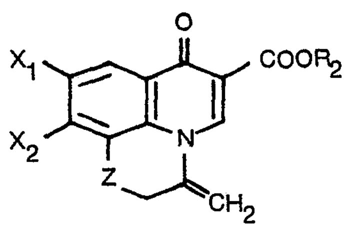

(II)

worin

$X_1$ und Z die oben angegebenen Bedeutungen besitzen,

$X_2$ ein Halogenatom bedeutet und

$R_2$ ein Wasserstoffatom oder eine Alkylgruppe bedeutet, mit einem cyclischen Amin der Formel

$$R_3—H$$

umsetzt, worin

$R_3$ eine Aminogruppe bedeutet, die Teil eines 4-7-gliedrigen cyclischen Rings darstellt, der gewünschtenfalls ein weiteres oder mehrere weitere N-, S- oder O-Atom(e) enthält und gewünschtenfalls substituiert ist durch einen oder mehrere Substituenten ausgewählt aus der Gruppe bestehend aus einer Hydroxygruppe, Alkylgruppe, Hydroxyalkylgruppe, Dialkylaminoalkylgruppe, geschützten Aminogruppe, geschützten Monoalkylaminogruppe und geschützten Aminoalkylgruppe,

wobei die oben aufgeführten Alkyleinheiten 1 bis 6 Kohlenstoffatome aufweisen,

gewünschtenfalls dann die Schutzgruppe entfernt und die Estereinheit hydrolisiert und

falls erforderlich die erhaltene Verbindung in ein physiolgisch verträgliches Salz davon überführt.

10. Pharmazeutisches Mittel, enthaltend mindestens eine der Verbindungen nach den Ansprüchen 1 bis 8 und einen pharmazeutisch verträglichen Träger.

**Patentanspruch für den Vertragsstaat: AT**

Verfahren zur Herstellung der Verbindungen der allgemeinen Formel (I)

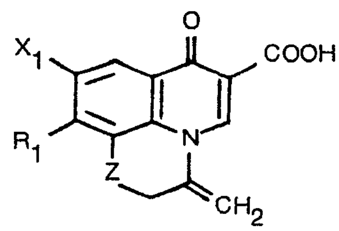

(I)

worin

$X_1$ ein Wasserstoffatom oder ein Halogenatom bedeutet,

Z ein Sauerstoffatom oder eine Methylengruppe bedeutet, und

$R_1$ eine Aminogruppe bedeutet, die Teil einer 4-7-gliedrigen cyclischen Gruppe darstellt, welche gewünschtenfalls ein zusätzliches oder mehrere zusätzliche N-, S- oder O-Atom(e) enthält und gewünschtenfalls substituiert ist durch einen oder mehrere Substituenten, ausgewählt aus der Gruppe bestehend aus einer Hydroxygruppe, Aminogruppe, Alkylgruppe, Mono- oder Dialkylaminogruppe, Hydroxyalkylgruppe und Aminoalkylgruppe,

wobei die oben genannten Alkyleinheiten 1 bis 6 Kohlenstoffatome aufweisen,

und die physiologisch verträglichen Salze davon, dadurch gekennzeichnet, daß man eine Verbindung der allgemeinen Formel (II)

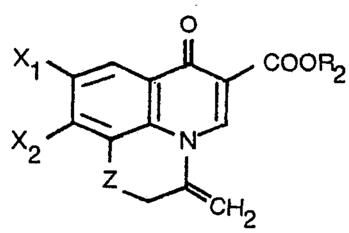

(II)

worin

$X_1$ und Z die oben angegebenen Bedeutungen besitzen,

$X_2$ ein Halogenatom bedeutet und

$R_2$ ein Wasserstoffatom oder eine Alkylgruppe bedeutet, mit einem cyclischen Amin der Formel

$$R_3—H$$

umsetzt, worin

$R_3$ eine Aminogruppe bedeutet, die Teil eines 4-7-gliedrigen cyclischen Rings darstellt, der gewünschtenfalls ein weiteres oder mehrere weitere N-, S- oder O-Atom(e) enthält und gewünschtenfalls substituiert ist durch einen oder mehrere Substituenten ausgewählt aus der Gruppe bestehend aus einer Hydroxygruppe, Alkylgruppe, Hydroxyalkylgruppe, Dialkylaminoalkylgruppe, geschützten Aminogruppe, geschützten Monoalkylaminogruppe und geschützten Aminoalkylgruppe,

wobei die oben aufgeführten Alkyleinheiten 1 bis 6 Kohlenstoffatome aufweisen,

gewünschtenfalls dann die Schutzgruppe entfernt und die Estereinheit hydrolisiert und

falls erforderlich die erhaltene Verbindung in ein physiolgisch verträgliches Salz davon überführt.

**Revendications pour les Etats contractants: BE CH DE FR GB IT LI NL SE**

1. Composés de formule générale (I):

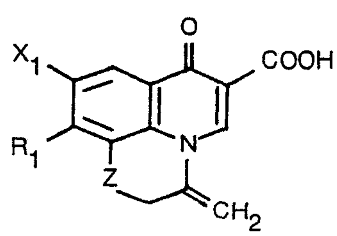

(I)

dans laquelle:

$X_1$ représente un atome d'hydrogéne ou un atome d'halogène,

Z représente un atome d'oxygène ou un groupe méthylène, et

$R_1$ représente un groupe amino qui fait partie d'un groupe cylique à 4—7 chaînons contenant factultativement un ou plusieurs autres atomes N, S ou O et facultativement subsitué par un ou plusieurs substituants choisi parmi les groupes hydroxyle, amino, alkyle, mono- ou dialkylamino, hydroxyalkyle et aminoalkyle

dans lesquels les restes alkyles mentionnés ci-dessus ont de 1 à 6 atomes de carbone

et leurs sels physiologiquement acceptables.

2. Composés selon la revendication 1, dans lesquels $X_1$ est un atome de fluor.

3. Composés selon la revendication 1, dans lesquels $X_1$ est un atome de fluor et $R_1$ est un groupe pyrrolidinyle substitué.

4. Composé selon la revendication 1, dans lequel Z est un atome d'oxygène, $X_1$ est un atome de fluor et $R_1$ est un groupe 3-hydroxy-1-pyrrolidinyle.

5. Composé selon la revendication 1, dans lequel Z est un atome d'oxygène, $X_1$ est un atome de fluor et $R_1$ est un groupe 3-amino-1-pyrrolidinyle.

6. Composé selon la revendication 1, dans lequel Z est un groupe méthylène, $X_1$ est un atome de fluor et $R_1$ est un groupe 3-hydroxy-1-pyrrolidinyle.

7. Composé selon la revendication 1, dans lequel Z est un groupe méthylène, $X_1$ est un atome de fluor et $R_1$ est un groupe 3-amino-1-pyrrolidinyle.

8. Composé selon la revendication 1, dans lequel Z est un atome d'oxygène, $X_1$ est un atome de fluor et $R_1$ est un groupe 4-méthyl-1-piperazinyle.

9. Procédé pour la fabrication des composés de formule générale (I) selon les revendications 1 à 8, caractérisé en ce que l'on fait réagir un composé de formule générale (II):

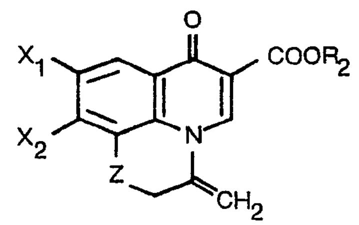

(II)

dans laquelle

$X_1$ et Z sont tels que définis ci-dessus,

$X_2$ représente un atome d'halogène, et

$R_2$ représente un atome d'hydrogène ou un groupe alkyle, avec une amine cyclique de formule $R_3$—H, dans laquelle

$R_3$ représente un groupe amino qui fait partie d'un noyau cyclique à 4—7 chaînons contenant facultativement ou un plusieurs autres atomes, N, S ou O et facultativement substitué par un ou plusieurs substituants choisis parmi les groupes hydroxyle, alkyle, hydroxyalkyle, dialkylamino, amino protégé, monoalkylamino protégé et aminoalkyle protégé

dans lesquels les restes alkyles mentionnés ci-dessus ont de 1 à 6 atomes de carbone,

ensuite, facultativement, on élimine le groupe protecteur et on hydrolyse le reste ester et, si on le désire, on transforme le composé obtenu en un de ses sels physiologiquement acceptables.

10. Composition pharmaceutique contenant ou moins un des composés selon les revendications 1 à 8 et un support pharmaceutiquement acceptable.

**Revendication pour l'Etat contractant: AT**

Procédé pour fabriquer les composés de formule générale (I):

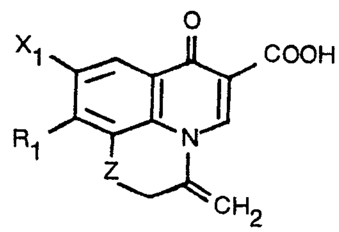

(I)

dans laquelle:

$X_1$ représente un atome d'hydrogène ou un atome d'halogène,

Z représente un atome d'oxygène ou un groupe méthylène, et

$R_1$ représente un groupe amino qui fait partie d'un groupe cylique à 4—7 chaînons contenant

facultativement un ou plusieurs autres atomes N, S ou O et facultativement substitué par un ou plusieurs substituants choisi parmi les groupes hydroxyle, amino, alkyle, mono- ou dialkylamino, hydroxyalkyle et aminoalkyle

dans lesquels les restes alkyles mentionnés ci-dessus ont de 1 à 6 atomes de carbone

et leurs sels physiologiquement acceptables, caractérisé en ce que l'on fait réagir un composé de formule générale (II):

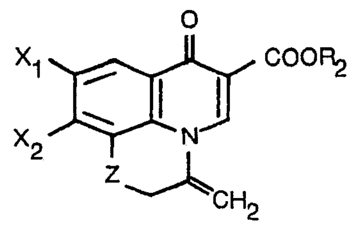

(II)

dans laquelle

$X_1$ et Z sont tels que définis ci-dessus,

$X_2$ représente un atome d'halogène, et

$R_2$ représente un atome d'hydrogène ou un groupe alkyle, avec une amine cyclique de formule $R_3$—H, dans laquelle

$R_3$ représente un groupe amino qui fait partie d'un noyau cyclique à 4—7 chaînons contenant facultativement ou un plusieurs autres atomes, N, S ou O et facultativement substitué par un ou plusieurs substituants choisis parmi les groupes hydroxyle, alkyle, hydroxyalkyle, dialkylamino, amino protégé, monoalkylamino protégé et aminoalkyle protégé

dans lesquels les restes alkyles mentionnés ci-dessus ont de 1 à 6 atomes de carbone,

ensuite, facultativement, on élimine le groupement protecteur et on hydrolyse le reste ester et, si on le désire, on transforme le composé obtenu en un de ses sels physiologiquement acceptables.